Europäisches Patentamt

**European Patent Office**

**Office européen des brevets**

(19)

(11) Numéro de publication: **0 140 787**

**B1**

(12) **FASCICULE DE BREVET EUROPÉEN**

(45) Date de publication du fascicule du brevet:
27.04.88

(51) Int. Cl.⁴: **G 01 N 33/553,** B 03 C 1/28,
H 01 F 7/04, B 25 B 11/00

(21) Numéro de dépôt: **84402116.2**

(22) Date de dépôt: **22.10.84**

(54) **Perfectionnements apportés aux moyens magnétiques destinés à retirer des billes de gel magnétique d'un fluide de dosage.**

(30) Priorité: **27.10.83 FR 8317166**

(43) Date de publication de la demande:
**08.05.85 Bulletin 85/19**

(45) Mention de la délivrance du brevet:
**27.04.88 Bulletin 88/17**

(84) Etats contractants désignés:
**AT BE CH DE FR GB IT LI LU NL SE**

(56) Documents cité:
**US-A-2 471 764**
**US-A-3 985 649**
**US-A-4 272 510**

(73) Titulaire: **INSTITUT PASTEUR Fondation reconnue d'utilité publique, 28 rue du Docteur Roux, F-75724 Paris Cédex 15 (FR)**

(72) Inventeur: **Daty, Jean, 398 Route de Chantilly, F-60520 La Chapelle en Serval (FR)**
Inventeur: **Assimon, André, 7 rue de Rethondes, F-95100 Argenteuil (FR)**

(74) Mandataire: **Ores, Irène, CABINET ORES 6, Avenue de Messine, F-75008 Paris (FR)**

LIBER, STOCKHOLM 1988

### Description

La présente invention est relative à des perfectionnements apportés aux moyens magnétiques destinés à retirer des billes de gel magnétique d'un milieu biologique.

Les gels magnétiques utilisables pour des dosages immunoenzymatiques sont connus par le Brevet INSTITUT PASTEUR 2 334 106. Ces gels magnétiques sont constitués par un gel d'acrylamide et/ou d'agarose contenant des particules magnétiques, couplé avec une protéine telle qu'un anticorps ou un antigène, par l'intermédiaire d'un agent de couplage approprié, tel que le glutaraldéhyde. Ils sont utilisés pour réaliser le dosage immunoenzymatique de l'antigène ou de l'anticorps correspondant contenu dans un liquide biologique à doser. Après incubation, le gel magnétique est séparé dans une première étape, du liquide biologique et, dans une seconde étape, de la protéine (antigène ou anticorps) marquée par une enzyme de dosage immunoenzymatique, chaque fois en appliquant un champ magnétique à l'extérieur du tube dans lequel a lieu le dosage, pour retenir le gel magnétique sur les parois du tube.

La demande de Brevet INSTITUT PASTEUR FR-A-82 20632 utilise ces gels magnétiques sur lesquels sont couplés des anticorps anti-déterminants antigéniques spécifiques, pour la détection immunobactériologique de germes pathogènes possédant des déterminants antigéniques spécifiques, dans des milieux biologiques contaminés. Conformément au procédé de détection qui fait l'objet de cette demande de brevet, après incubation avec le liquide biologique supposé contaminé, les billes de gel magnétique sont retirées du liquide biologique par des moyens magnétiques, de préférence constitués par une tige aimantée, à l'aide desquels ils sont transférés sur un milieu gélosé sur lequel le processus de détection est poursuivi. La tige aimantée utilisée à cet effet est une tige aimantée enrobée de "Téflon".

Il est cependant apparu qu'une telle tige aimantée présente l'inconvénient d'une part d'être insuffisamment isolée par l'enrobage de "Téflon" et d'autre part d'être susceptible d'entrer en contact indésirable avec des surfaces naturellement magnétiques au cours de l'opération de transfert.

Par ailleurs, il est décrit dans le Brevet USA-4 272 510 un procédé pour transférer simultanément une pluralité d'unités magnétiques adsorbant à leur surface des antigènes ou des anticorps, ledit transfert étant effectué par attraction magnétique au moyen d'un dispositif sous forme de sondes en matériau magnétique.

D'autre part, le Brevet US-A-2 471 764 décrit un instrument manuel, constitué par un dispositif comprenant un aimant, pour ramasser de petits objets en matériau magnétique, le ramassage ne pouvant s'effectuer que quand l'aimant est maintenu manuellement en-dehors du dispositif où il est logé.

La présente invention s'est en conséquence donné pour but de pourvoir à un dispositif aimanté qui répond mieux aux nécessités de la pratique que les moyens magnétiques visant aux mêmes buts, antérieurement connus, notamment en ce qu'il est pourvu d'une isolation qui évite toute fausse manoeuvre et de moyens de rappel qui empêchent un contact accidentel des moyens magnétiques avec des surfaces magnétiques pendant le transfert des billes de gel magnétique.

La présente invention a pour objet un dispositif aimanté destiné à retirer des billes de gel magnétique ou analogues d'un fluide biologique et à les transférer sur un milieu de dosage immunoenzymatique, du type comportant une tige aimantée, lequel dispositif est illustré par le dessin, qui est expliqué ci-dessuos et est caractérisé en ce qu'il comprend: - un boîtier en matériau isolant, notamment en matière plastique, dans lequel est ménagé un orifice traversant sur toute la hauteur dudit boîtier, lequel boîtier contient un aimant - une tige logée dans ledit orifice traversant qui constitue une voie de guidage de ladite tige, laquelle tige est formée d'une partie inférieure et d'une partie supérieure, la partie inférieure de la tige étant réalisée en matériau magnétique et la partie supérieure étant réalisée en matériau non magnétique, ladite partie supérieure étant emboîtée dans la partie inférieure et maintenue dans cette dernière à l'aide d'un cliquet ou analogue, - une rondelle de butée fixée à la face inférieure du boîtier, à la sortie de l'orifice traversant susdit, - un ressort monté entre une rondelle de butée fixée au voisinage de l'extrémité supérieure de la tige et, de façon spécifique, au voisinage de l'extrémité de la partie supérieure, en matériau non magnétique, de ladite tige, et la face supérieure du boîtier.

Selon un autre mode de réalisation avantageux du dispositif aimanté conforme à l'invention, le boîtier est réalisé sous forme de poignée isolante dudit dispositif.

Selon encore un autre mode de réalisation avantageux du dispositif aimanté conforme à l'invention, l'orifice traversant de guidage de ladite tige est excentré par rapport à l'axe longitudinal du boîtier.

La présente invention a également pour objet un ensemble ou "kit" prêt à l'emploi pour la réalisation de dosages immunoenzymatiques, caractérisé en ce qu'il comprend
- un réceptacle contenant une quantité appropriée de billes de gel magnétique couplé avec un antigène ou un anticorps spécifique,
- un dispositif aimanté tel que défini dans ce qui précède,
- une boîte de culture, telle qu'une boîte de Petri notamment, dont la face inférieure est pourvue extérieurement d'un aimant de forte puissance,
- un réceptacle contenant une quantité appropriée de milieu de culture pour la culture de germes pathogènes à doser, destiné à être introduit dans ladite boîte de culture,

- un réceptacle contenant une quantité appropriée d'un anticorps ou d'un antigène spécifique anti-germe pathogène à détecter, destiné à être introduit sur le milieu de culture susdit,

- un réceptacle contenant une quantité appropriée d'agents de lyse des cultures de germes pathogènes développées dans ladite boîte de culture,

- un réceptacle contenant une quantité appropriée d'eau de lavage physiologique tamponnée pour lavage des billes de gel magnétique fixées sur la tige du dispositif aimanté susdit.

Outre les dispositions qui précèdent, l'invention comprend encore d'autres dispositions, qui ressortiront de la description qui va suivre.

L'invention vise plus particulièrement les dispositifs aimantés conformes aux dispositions qui précèdent et les ensembles de dosage immunoenzymatique incluant de tels dispositifs.

L'invention sera mieux comprise à l'aide du complément de description qui va suivre, qui se réfère au dessin annexé dans lequel:

- la fig. 1 est une vue de face du dispositif aimanté conforme à l'invention, rappelé en position de transfert des billes de gel magnétique qui sont fixées sur la tige aimantée,

- la fig. 2 est une vue de dessus du dispositif de la fig. 1 et

- la fig. 3 est une vue de face du dispositif de la fig. 1 en position de détachement des billes sur un milieu de dosage approprié.

Il doit être bien entendu, toutefois, que ce dessin et les parties descriptives correspondantes, sont donnés uniquement à titre d'illustration de l'objet de l'invention, dont ils ne constituent en aucune manière une limitation.

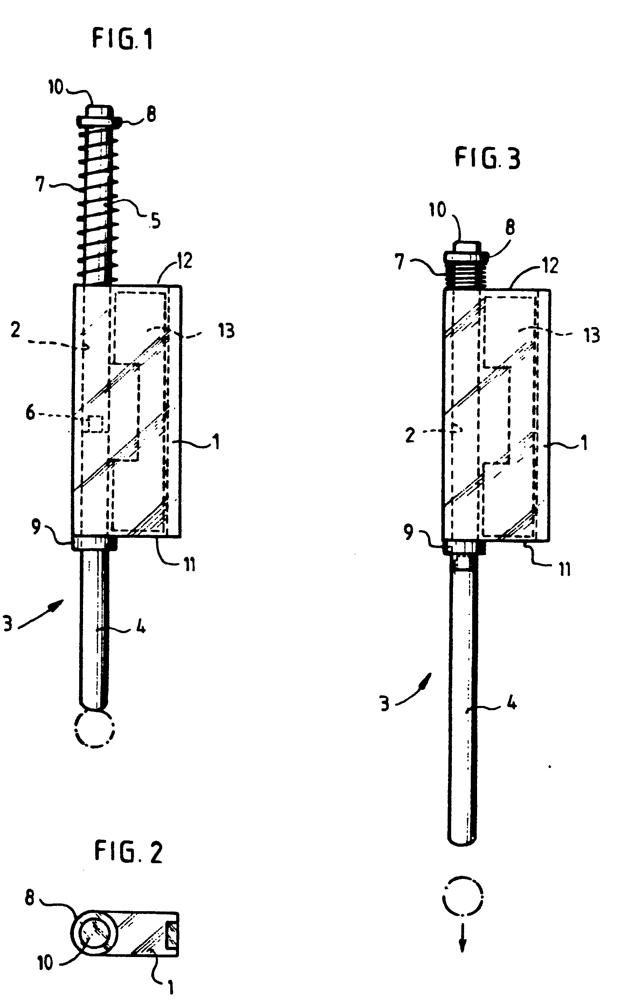

Le dispositif aimanté représenté aux fig. 1 à 3, comprend un boitier 1 dans lequel est ménage un orifice traversant 2, qui s'étend sur la totalité de la hauteur du boitier 1. Bien que l'emplacement de l'orifice traversant puisse être quelconque, néanmoins, pour des raisons de commodité, dans la mesure où le boitier, qui est réalisé en matériau isolant, est utilisé comme poignée de préhension du dispositif conforme à l'invention, il est préféré que l'orifice traversant 2 soit excentré par rapport à l'axe longitudinal du boitier.

Une tige, désignée d'une façon générale par la référence 3, dont le diamètre est sensiblement égal à celui de l'orifice traversant 2, est montée dans l'orifice traversant 2, de manière à pouvoir coulisser dans ce dernier. Cette tige 3 comprend une partie inférieure 4 en matériau magnétique et une partie supérieure 5 en matériau non magnétique ; la partie supérieure 5 de la tige 3 est emboîtée dans l'extrémité supérieure de la partie inférieure 4, dans laquelle elle est fixée en position par un cliquet 6, ou par tout autre système de fixation approprié, tel que, par exemple, un système à baïonnette.

Un ressort 7 est monté sur la tige 3, entre une rondelle de butée 8 fixée au voisinage de l'extrémité supérieure 10 de la tige 3, et la face supérieure 12 du boîtier 1. Une rondelle de butée 9 est fixée à la sortie de l'orifice traversant 2, sur la face inférieure 11 du boîtier 1. Le boîtier 1 contient un aimant 13.

Le fonctionnement du dispositif aimanté conforme à l'invention est le suivant : une pression exercée sur l'extrémité supérieure 10 de la tige 3 provoque le coulissement de la tige 3 vers le bas, dans l'orifice traversant 2 ménagé dans le boîtier 1, la course de la tige 3 étant limitée par le ressort 7 et par la compression concommittante de ce dernier entre la butée 8 et la face supérieure 12 du boîtier 1 (cf. figure 3). Le relâchement de la poussée exercée sur l'extrémité supérieure 10 de la tige 3 provoque le rappel de la tige 3 par le ressort 7, dans la position représentée à la figure 1, dans laquelle la partie 4 de la tige est aimantée par l'aimant 13 contenu dans le boîtier 1.

Dans son application aux procédés de dosage objets des Brevets FR-A-2 334 106 et 82 20632, le dispositif aimanté conforme à l'invention est placé au-dessus d'un récipient contenant un milieu d'incubation comprenant un fluide biologique et des billes de gel magnétique couplées à une protéine appropriée pour permettre la réalisation d'un dosage immunoenzymatique ; une pression du doigt exercée par un opérateur sur l'extrémité supérieure 10 de la tige 3, met en contact la partie inférieure 4 aimantée de la tige 3 avec le milieu, laquelle attire les billes de gel magnétique, qui s'y fixent. Le relâchement de la pression exercée sur l'extrémité supérieure 10 de la tige 3, provoque le rappel de la tige 3 dans la position représentée à la fig. 1, et la mise de la partie inférieure 4 de la tige 3 hors de contact avec le milieu. Les billes de gel magnétique fixées sur la partie 4 de la tige 3 sont transportées alors que le dispositif se trouve dans la position représentée à la fig. 1, jusqu'à un récipient contenant un milieu de dosage approprié. Une nouvelle pression est alors exercée sur l'extrémité supérieure 10 de la tige 3 par l'opérateur pour mettre en contact la partie inférieure 4 de la tige 3 avec le milieu de dosage qui est contenu dans un récipient approprié pourvu extérieurement d'un aimant dont la force d'attraction détache les billes de gel magnétique de la partie 4 de la tige 3 pour les faire tomber sur le milieu de dosage.

La pression exercée sur l'extrémité 10 de la tige 3 est alors relâchée, ce qui provoque le rappel de la tige 3 en position de repos (représentée à la fig. 1), le dispositif aimanté conforme à l'invention étant prêt pour une nouvelle opération.

Le dispositif aimanté conforme à l'invention est parfaitement isolé grâce à la disposition du boîtier 1 et à la réalisation de la tige 3 en deux parties dont la partie supérieure est non magnétique. De plus, tout contact accidentel de la partie aimantée de la tige avec des surfaces aimantées est empêché dans une grande mesure par le rappel de la tige 3 en position haute pendant son transfert du récipient contenant le milieu d'incubation au récipient contenant le milieu de dosage.

**Revendications**

1. Dispositif aimanté destiné à retirer des billes de gel magnétique ou analogues d'un fluide biologique et à les transférer sur un milieu de dosage immunoenzymatique, du type comportant une tige aimantée, lequel dispositif est caractérisé en ce qu'il comprend: - un boîtier (1) en matériau isolant, notamment en matière plastique, dans lequel est ménagé un orifice traversant (2) sur toute la hauteur dudit boîtier, lequel boîtier contient un aimant (13) - une tige (3) logée dans ledit orifice traversant (2) qui constitue une voie de guidage de ladite tige, laquelle tige (3) est formée d'une partie inférieure (4) et d'une partie supérieure (5), la partie inférieure (4) de la tige (3) étant réalisée en matériau magnétique et la partie supérieure (5) étant réalisée en matériau non magnétique, ladite partie supérieure (5) étant emboîtée dans la partie inférieure (4) et maintenue dans cette dernière à l'aide d'un cliquet (6) ou analogue,
- une rondelle de butée (9) fixée à la face inférieure (11) du boîtier, à la sortie de l'orifice traversant (2) susdit,
- un ressort (7) monté entre une rondelle de butée (8) fixée au voisinage de l'extrémité supérieure (10) de la tige (3) et, de façon spécifique, au voisinage de l'extrémité de la partie supérieure (5), en matériau non magnétique, de ladite tige (3), et la face supérieure (12) du boîtier.

2. Dispositif aimanté selon la revendication 1, caractérisé en ce que le boîtier (1) est réalisé sous forme de poignée isolante dudit dispositif.

3. Dispositif aimanté selon l'une quelconque des revendications 1 à 2, caractérisé en ce que l'orifice traversant (2) de guidage de ladite tige (3) est excentré par rapport à l'axe vertical du boîtier (1).

4. Ensemble ou "kit" prêt à l'emploi pour la réalisation de dosages immunoenzymatiques, caractérisé en ce qu'il comprend :
- un réceptacle contenant une quantité appropriée de billes de gel magnétique couplé avec un antigène ou un anticorps spécifique,
- un dispositif aimanté selon l'une quelconque des revendications 1 à 3,
- une boîte de culture, telle qu'une boîte de Petri notamment, dont la face inférieure est pourvue extérieurement d'un aimant de forte puissance,
- un réceptacle contenant une quantité appropriée de milieu de culture pour la culture de germes pathogènes à doser, destiné à être introduit dans ladite boîte de culture,
- un réceptacle contenant une quantité appropriée d'un anticorps ou d'un antigène spécifique antigerme pathogène à détecter, destiné à être introduit sur le milieu de culture susdit,
- un réceptacle contenant une quantité appropriée d'agents de lyse des cultures de germes pathogènes développées dans ladite boîte de culture,
- un réceptacle contenant une quantité appropriée d'eau de lavage physiologique tamponnée.

**Patentansprüche**

1. Magnetvorrichtung zum Herausziehen von Kugeln aus magnetischem Gel oder ähnlichem aus einer biologischen Flüssigkeit und Übertragen der Kugeln auf ein immunenzymatisches Dosierungsmedium, umfassend einen magnetisierten Stab, wobei die Vorrichtung gekennzeichnet ist durch:
ein Gehäuse (1) aus isolierndem Material, insbesondere Kunststoffmaterial, in dem eine durchgehende Öffnung (2) über die gesamte Höhe des Gehäuses ausgespart ist, und das Gehäuse einen Magneten (13) enthält,
einen Stab (3), der in der durchgehenden Öffnung (2) angeordnet ist, die eine Führung für den Stab bildet, der Stab (3) aus einem unteren Teil (4) und einem oberen Teil (5) gebildet ist, wobei der untere Teil (4) des Stabes (3) aus einem magnetischen Material und der obere Teil (5) aus einem nicht-magnetischen Material besteht, der obere Teil (5) in dem unteren Teil (4) eingefügt und mittels einer Sperrklinke (6) oder ähnlichem darin gehalten ist,
eine Anschlagscheibe (9), die an der unteren Fläche (11) des Gehäuses am Ausgang der durchgehenden Öffnung (2) befestigt ist, eine Feder (7), die zwischen einer Anschlagscheibe (8), die nahe dem oberen Endstück (10) des Stabes (3) und in spezifischer Weise nahe dem Endstück des oberen Teiles (5) aus nichtmagnetischem Material des Stabes (3) und der oberen Fläche (12) des Gehäuses montiert ist.

2. Magnetvorrichtung nach Anspruch 1, dadurch gekennzeichnet, daß das Gehäuse (1) als isolierender Handgriff der Vorrichtung ausgebildet ist.

3. Magnetvorrichtung nach einem der Ansprüche 1 bis 2, dadurch gekennzeichnet, daß die eine Führung für den Stab (3) bildende durchgehende Öffnung (2) bezüglich der Vertikalachse des Gehäuses (1) exzentrisch angeordnet ist.

4. Kombination zur Verwendung für immunenzymatische Dosierungen, gekennzeichnet durch:
einen Behälter, der eine geeignete Menge von Kugeln aus magnetischem Gel enthält, die mit einem spezifischen Antigen oder Antikörper gekuppelt sind,
eine Magnetvorrichtung gemäß irgendeinem der Ansprüche 1 bis 3, eine Kulturschale, insbesondere eine Petrischale, deren untere Fläche außen mit einem starken Magneten versehen ist,

einen Behälter, der eine geeignete Menge des Kulturmediums zum Züchten der zu dosierenden pathogenen Keime enthält, das zum Einführen in die genannte Kulturschale bestimmt ist,

einen Behälter, der eine geeignete Menge eines Antikörpers oder eines spezifisch gegen die nachzuweisenden pathogenen Keime wirkenden Antigens enthält, bestimmt, auf das genannte Kulturmedium überführt zu werden,

einen Behälter, der eine geeignete Menge von Auflösungsmitteln für die Kulturen der pathogenen Keime enthält, die sich in der genannten Kulturschale entwickelt haben und

einen Behälter, der eine geeignete Menge tamponierten physiologischen Waschwassers enthält.

## Claims

1. Magnetic means for the removal of magnetic gel beads or the like from a biological fluid and for transferring them to an immunoenzymatic assay medium, of the type comprising a magnetised rod, said means comprising a box of insulating material, particularly of plastics material, in which it formed an orifice traversing the whole height of said box, said box containing a magnet; a rod housed in said traversing orifice which constitutes a guide path for said rod, said rod being formed by a lower part and an upper part, said lower part of the rod being formed of a magnetic material and said upper part being formed of a non-magnetic material, said upper part being fitted into said lower part and held in the latter by means of a catch or the like; a stop ring fixed to the lower surface of the box, at the exit of the above said traversing orifice; a spring mounted between a stop ring fixed in the vicinity of the upper end of the rod and, specifically, in the vicinity of the end of the upper part, of non-magnetic material of said rod, and the upper surface of the box.

2. Magnetic means according to claim 1, wherein the box is constructed in the form of an insulating handle for said means.

3. Magnetic means according to claim 1 wherein said traversing orifice for guiding said rod is excentric with respect to the vertical axis of the box.

4. Ready- for -use outfit or "kit" for performing immunoenzymatic assays, said "kit" comprising:
- a receptacle containing a suitable amount of beads of magnetic gel coupled with an antigen or a specific antibody,
- a magnetic means according to claim 1,
- a culture dish, such as particularly a Petri dish, whose upper surface is provided externally with a high powered magnet,
- a receptacle containing a suitable amount of culture medium for the culture of pathogenic germs to be assayed, designed to be introduced into said culture dish,
- a receptacle containing a suitable amount of an antibody or of a pathogenic specific antigerm antigen, designed to be introduced onto said culture medium,
- a receptacle containing a suitable amount of agents for the lysis of pathogenic germ cultures developed in said culture dish,
- a receptacle containing a suitable amount of buffered physiological washing liquid.

FIG.1

FIG.3

FIG. 2